# EUROPEAN PATENT SPECIFICATION

(11) **EP 3 482 695 B1**
(45) Date of publication and mention of the grant of the patent: **22.03.2023**
(21) Application number: 18205075.7
(22) Date of filing: 08.11.2018
(51) Int. Cl.: A61B 17/064, A61B 17/072

(54) **SURGICAL STAPLES WITH EXPANDABLE BACKSPAN**
CHIRURGISCHE KLAMMERN MIT ERWEITERBARER RÜCKENBREITE
AGRAFES CHIRURGICALES À BARRE TRANSVERSALE EXTENSIBLE

(30) Priority: 09.11.2017 US 201715808203
(43) Date of publication of application: 15.05.2019
(73) Proprietor: Covidien LP, Mansfield, MA 02048 (US)
(72) Inventor: Kostrzewski, Stanislaw, Newtown, CT 06470 (US)
(74) Representative: Maschio, Antonio

(56) References cited:
- EP-A2- 3 257 448
- WO-A1-89/04146
- WO-A1-2011/153408
- WO-A2-2012/031204
- US-A1- 2008 172 088

## Description

### CROSS-REFERENCE TO RELATED APPLICATION

This application is a continuation in part of U.S. Patent Application No. 15/587,747 filed May 5, 2017.

### BACKGROUND

### 1. Technical Field

The present disclosure relates to surgical staples for use with surgical stapling instruments. More particularly, the present disclosure relates to surgical staples for use with surgical stapling instruments for joining tissue of varying thicknesses.

### 2. Background Of Related Art

Surgical staples are applied to tissue using surgical stapling instruments to join tissue or tissue segments in a fast and efficient manner in a variety of surgical procedures, e.g., anastomoses procedures.

Typically, a surgical staple includes a backspan and a pair of spaced legs. The legs are driven through tissue and into an anvil assembly of a surgical stapling instrument to deform the staple into a desired configuration, e.g., B-staple, to effect hemostasis. Current surgical staples are particularly sized and suited for tissue of a given thickness range to effect hemostasis. As such, a clinician must choose a staple having an appropriate size for a given tissue thickness range to ensure effective hemostasis. If the tissue thickness is misidentified by the clinician or if the tissue thickness falls near the outer edges of the range for a given staple size, the likelihood of ineffective hemostasis is increased.

Accordingly, a continuing need exists in the suturing arts for a surgical staple that is capable of accommodating a greater range of tissue thicknesses to provide the clinician greater flexibility when performing a variety of surgical procedures.

US 2008/172088 A1 relates to a staple capable of placing a load-bearing force against the material being stapled and improvements in processes for stapling material.

### SUMMARY

The present disclosure provides in one aspect a surgical staple including a body having a diameter and including a first leg, a second leg, and a backspan. The first and second legs each have a first end portion and a second end portion. The backspan has a first portion, a second portion, a third portion, and a crimped region. The first portion of the backspan includes a looped member having a first end portion and a second end portion. The second portion of the backspan extends between the first end portion of the first leg and the first end portion of the looped member. The third portion of the backspan extends between the first end portion of the second leg and the second end portion of the looped member. The crimped region includes a crimped section defined in at least one of the second portion or the third portion of the backspan. A combined wire thickness of the second and third portions of the backspan in the crimped region is about equal to the diameter of the body of the surgical staple.

According to the invention the crimped region of the backspan includes a first crimped section defined on the second portion of the backspan and a second crimped section defined on the third portion of the backspan. In such embodiments, the combined wire thickness of the crimped region is about equal to the diameter of the body of the surgical staple.

According to the invention the first crimped section of the second portion of the backspan overlaps with the second crimped section of the third portion of the backspan.

In certain examples, the first crimped section of the second portion of the backspan is axially aligned with the second crimped section of the third portion of the backspan.

In some examples, the looped member of the backspan defines a first axis that extends between the first end portion of the looped member and the second end portion of the looped member.

In certain examples, the second portion of the backspan defines a second axis and the third portion of the backspan defines a third axis. In such embodiments, the first axis of the looped member is parallel with the second axis of the second portion and the third axis of the third portion.

In some examples, the second axis of the second portion of the backspan is laterally aligned with the third axis of the third portion of the backspan such that the first leg and the second leg of the surgical staple are axially aligned.

In certain examples, the looped member of the backspan includes an apex, the second portion of the backspan includes a first mid-portion, and the third portion of the backspan includes a second mid-portion. In such examples, the apex of the looped member and the first and second mid-portions of the second and third portions of the backspan define an axis that is perpendicular to the second axis of the second portion of the backspan and the third axis of the third portion of the backspan.

In some examples, the surgical staple includes an unformed configuration and a formed configuration. In the unformed configuration of the surgical staple, the apex of the looped member of the backspan is spaced apart from each of the second and third portions of the backspan a first distance. In the formed configuration of the surgical staple, the apex of the looped member of the backspan is spaced apart from each of the second and third portions of the backspan a second distance that is less than the first distance.

In some examples, the backspan of the surgical staple is positioned to engage tissue such that the second distance between the apex of the looped member of the backspan and each of the second and third portions of the backspan decreases as a thickness of tissue engaged by the backspan increases.

In certain examples, in the formed configuration of the surgical staple, the first leg of the surgical staple is positioned on a first lateral side of the looped member and the second leg of the surgical staple is positioned on a second lateral side of the looped member opposite the first lateral side of the looped member.

The present disclosure provides in another aspect a surgical staple including a body having a first leg, a second leg, and a backspan. The first and second legs each have a first end portion and a second end portion. The backspan has a first portion, a second portion, a third portion, and a crimped region. The first portion of the backspan has a first end portion and a second end portion. The second portion of the backspan extends longitudinally between the first end portion of the first leg and the first end portion of the first portion. The second portion defines a first axis. The third portion of the backspan extends longitudinally between the first end portion of the second leg and the second end portion of the first portion. The third portion defines a second axis. The crimped region includes a crimped section defined in at least one of the second portion or the third portion of the backspan. The crimped region aligns the first axis of the second portion with the second axis of the third portion.

In some examples, the first portion includes a looped member positioned between the first end portion and the second end portion of the first portion of the backspan.

In certain examples, the looped member of the first portion of the backspan defines a third axis that is parallel with the first axis of the second portion of the backspan and the second axis of the third portion of the backspan.

In some examples, in the crimped region, the second and third portions of the backspan include a combined wire thickness that is about equal to a diameter of the body of the surgical staple.

In some examples, the crimped section defined in the at least one of the second portion or the third portion of the backspan axially aligns the first end portion of the first leg with the first end portion of the second leg.

In certain examples, the crimped region of the backspan is disposed between the first end portion of the first leg and the first end portion of the second leg.

In certain examples, the second portion of the backspan and the third portion of the backspan are in lateral contact.

### BRIEF DESCRIPTION OF THE DRAWINGS

Various examples of the presently disclosed expandable backspan staples and cartridges for supporting the staples are disclosed herein with reference to the drawings, wherein:
FIG. 1 is a perspective view of an exemplary example of the presently disclosed surgical staple, illustrating the surgical staple in a unformed configuration;
FIG. 2 is a front view of the surgical staple of FIG. 1;
FIG. 3 is a top view of the surgical staple of FIG. 1;
FIG. 4A is a front view of the surgical staple of FIG. 1 in a formed configuration engaging tissue having a first thickness;
FIG. 4B is a front view of the surgical staple of FIG. 1 in the formed configuration engaging tissue having a second larger thickness;
FIG. 4C is a front view of the surgical staple of FIG. 1 in the formed configuration engaging tissue having a third even larger thickness;
FIG. 5 is a perspective view of a surgical stapling instrument including a cartridge that supports the surgical staples of FIG. 1 with a tool assembly of the surgical stapling instrument in an unclamped position;
FIG. 6 is a perspective view of a loading unit of the surgical stapling instrument of FIG. 5 with the tool assembly of the loading unit in the unclamped position;
FIG. 7 is a perspective view of the tool assembly of FIG. 6 in the clamped position;
FIG. 8 is an exploded view of a staple cartridge assembly of the loading unit of FIG. 6;
FIG. 9 is a top view of a staple cartridge of the staple cartridge assembly of FIG. 8;
FIG. 10 is an enlarged view of the indicated area of detail delineated as 10 in FIG. 9;
FIG. 11 is an enlarged view of the indicated area of detail delineated as 11 in FIG. 8;
FIG. 12 is a side, perspective view of a pusher of the staple cartridge assembly of FIG. 8;
FIG. 13 is a side, perspective view of the pusher of FIG. 12 with a plurality the surgical staples of FIG. 1 disposed on the pusher;
FIG. 14 is a top view of the pusher of FIG. 12;
FIG. 15 is a cross-sectional view taken along section lines "15-15" of FIG. 7 with tissue having a first thickness clamped within the tool assembly of the loading unit; and
FIG. 16 is a cross-sectional view taken along section lines "16-16" of FIG. 7 with tissue having a second thickness clamped within the tool assembly of the loading unit;
FIG. 17A is a perspective view of a surgical staple according to the present invention illustrating the surgical staple in a unformed configuration;
FIG. 17B is a top view of the surgical staple of FIG. 17A;
FIG. 17C is a bottom view of the surgical staple of FIG. 17A;
FIG. 17D is a side view of the surgical staple of FIG. 17A;
FIG. 18 is a side, perspective view of a pusher of a staple cartridge assembly in accordance with an aspect of the present disclosure that supports the surgical staples of FIG. 17A;
FIG. 19A is a side, perspective view of a staple cartridge assembly in accordance with another aspect of the present disclosure that supports the surgical staples of FIG. 17A; and
FIG. 19B is a side, perspective view of a staple cartridge of the staple cartridge assembly of FIG. 19A.

### DETAILED DESCRIPTION OF EMBODIMENTS

Examples of the presently disclosed surgical staple will now be described in detail with reference to the drawings wherein like references numerals identify similar or identical elements. In the drawings, and in the following description, the term "proximal" should be understood to refer to that portion or end of the instrument that is closest to a user during proper use, while the term "distal" should be understood to refer to that portion or end of the instrument that is furthest from a user during proper use, as is traditional and conventional in the art. Also, as used in the specification, the terms "approximately equal," "substantially equal," and "about equal" should be understood to include +/- 15% of a given parameter.

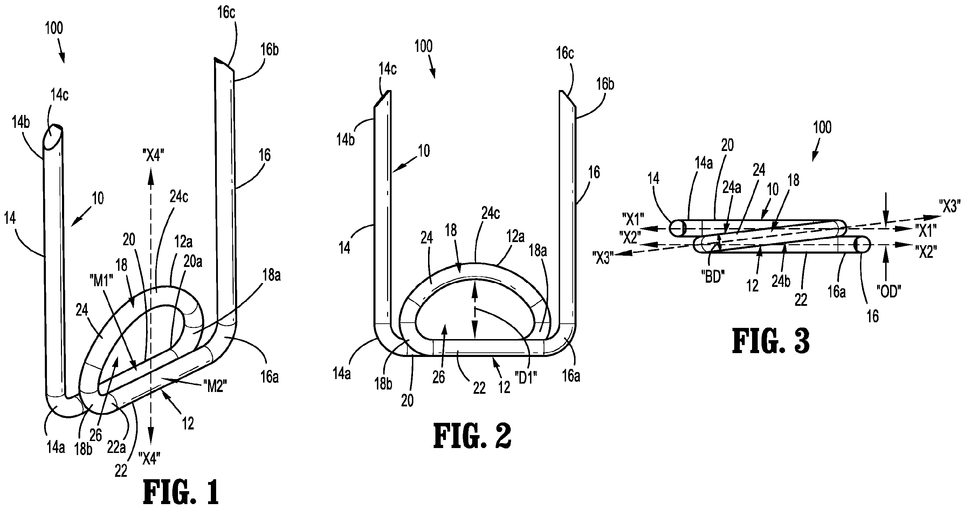

Referring initially to FIGS. 1-3, an example of the present disclosure is shown generally as a surgical staple 100. The surgical staple 100 includes a body 10 having a backspan 12, a first leg 14, and a second leg 16. The surgical staple 100 has an unformed configuration, as shown in FIGS. 1-3, wherein the first leg 14 and the second leg 16 are parallel, or substantially parallel, to one another and spaced a distance from one another. Alternatively, in the unformed configuration, the first and second legs 14, 16 can diverge slightly or converge slightly, etc. in relation to each other.

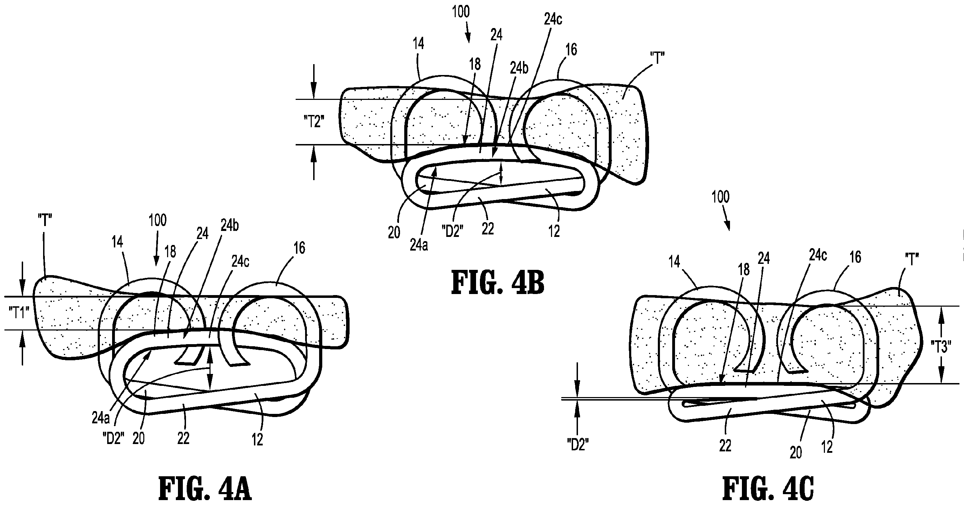

Each of the first and second legs 14, 16 includes a first end portion 14a, 16a, respectively, and a second end portion 14b, 16b, respectively. Each of the second end portions 14b, 16b of the first and second legs 14, 16 includes a tissue-penetrating tip 14c, 16c. In one embodiment, each of the tissue-penetrating tips 14c, 16c of the respective first and second legs 14, 16 of the surgical staple 100 can be formed with beveled or tapered end to facilitate penetration of the first and second legs 14, 16 into tissue "T" (see FIGS. 4A-4C). Alternately, the tissue penetrating tips 14c, 16c of the respective first and second legs 14, 16 of the surgical staple 100 need not be tapered, can be tapered in a different direction, or can define a conical or flat surface.

With continued reference to FIGS. 1-3, the backspan 12 includes a first portion 12a having a looped member 18, a second portion 20 that is substantially linear and extends between the first end portion 14a of the first leg 14 and a first end portion 18a of the looped member 18, and a third portion 22 that is substantially linear and extends between the first end portion 16a of the second leg 16 and a second end portion 18b of the looped member 18. The looped member 18 of the backspan 12 includes an arcuate portion 24 that extends between the second and third portions 20, 22 of the backspan 12. In the unformed configuration of the surgical staple 100, the second portion 20 of the backspan 12 may be substantially parallel to the third portion 22 of the backspan 12 and in lateral contact or close alignment with the third portion 22 of the backspan 12, as illustrated in FIG. 1.

In examples, the arcuate portion 24 of the looped member 18 extends transversely between the second portion 20 and the third portion 22 of the backspan 12. In the unformed configuration of the surgical staple 100, the second portion 20 of the backspan 12 defines an axis "X1-X1" and the third portion 22 of the backspan 12 defines an axis "X2-X2". The axis "X1-X1" of the second portion 20 and the axis "X2-X2" of the third portion 22 may be laterally offset from each other an offset distance "OD". It is envisioned that the offset distance "OD" between the axis "X1-X1" of the second portion 20 and the axis "X2-X2" of the third portion 22 may be substantially equal to a diameter "BD" of the body 10 of the surgical staple 100 such that the second portion 20 and the third portion 22 are closely positioned or in contact with each other.

The arcuate portion 24 of the looped member 18 defines an axis "X3-X3" that extends between a first end portion 20a of the second portion 20 of the backspan 12 and a first end portion 22a of the third end portion 22 of the backspan 12. In examples, the looped member 18 and the second and third portions 20, 22 may define an enclosed opening 26 which may have a circular or oval configuration. It is envisioned that the arcuate portion 24 of the looped member 18 of the backspan 12 can be formed having any desired radius of curvature to suit a particular need, surgical procedure, or range of tissue thicknesses (as will be discussed below). It is also envisioned that the opening 26 defined by the backspan 12 need not be circular or oval but rather may have other configurations, such as, for example, U-shaped, trapezoidal, rectangular, etc. The backspan can have other shapes, such as round, rectilinear, etc., and the member 18 can be straight, angled or curved. Further, in examples, the backspan 12 may include more than one looped member 18.

The body 10 of the surgical staple 100 can have a circular cross-section throughout its length. Alternatively, it is envisioned that the body 10 of the surgical staple 100 may have a variety of different cross-sectional shapes including rectangular, oval, square, triangular, trapezoidal, etc. It is also envisioned that the backspan 12 of the surgical staple 100 and the first and second legs 14, 16 of the surgical staple 100 may have different cross-sectional shapes. For example, in one example, the backspan 12 of the surgical staple 100 can have a rectangular cross-sectional shape and the first and second legs 14, 16 of the surgical staple 100 can have an oval cross-sectional shape.

The surgical staple 100 may be fabricated from a formable material, such as, for example, titanium, stainless steel or a variety of different bio-compatible polymers. In this manner, the surgical staple 100 may be introduced over tissue while in an unformed configuration, and then deformed or fastened onto the tissue to secure the surgical staple 100 to the tissue. It is contemplated that the surgical staple 100 may be fabricated from any nondegradable, biocompatible material known by those having skill in the art.

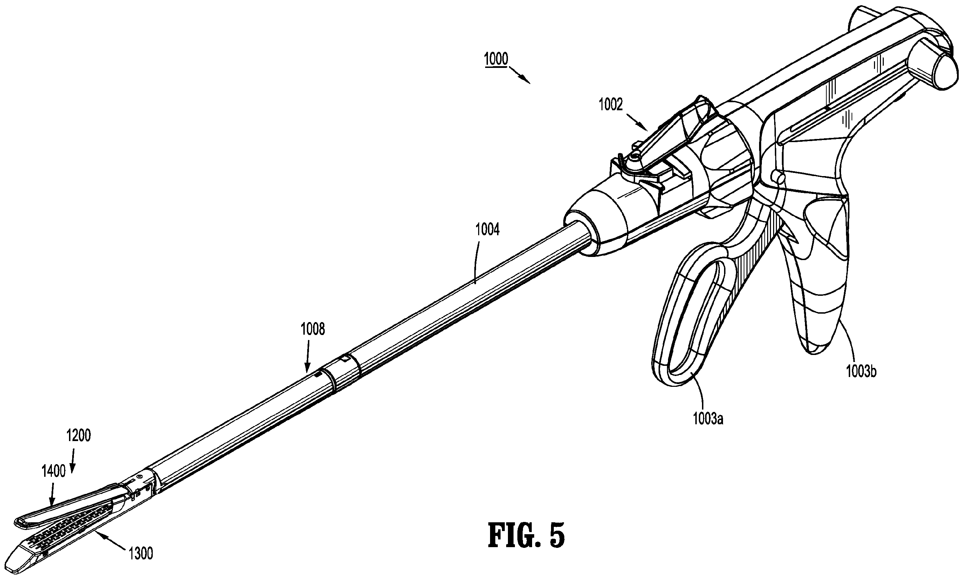

Referring now to FIGS. 4A-4C, in some examples, the first and second legs 14, 16 of the surgical staple 100 are deformed against an anvil assembly 1400 (see FIG. 5) of a surgical stapling instrument 1000 into a substantially B-shaped staple configuration. Since the first and second legs 14, 16 of the surgical staple 100 extend from the second and third portions 20, 22 of the backspan 12, respectively, and the axis "X1-X1" of the second portion 20 and the axis "X2-X2" of the third portion 22 are laterally offset from each other by the offset distance "OD," which is substantially equal to the diameter "BD" of the body 10 of the surgical staple 100, it is envisioned that in the formed configuration of the surgical staple 100, the first leg 14 of the surgical staple 100 is positioned on a first lateral side 24a of the arcuate portion 24 of the looped member 18 and the second leg 16 is positioned on a second, opposite lateral side 24b of the arcuate portion 24 of the looped member 18 (see FIGS. 3, 4A, and 4B).

In the formed configuration, the arcuate portion 24 of the looped member 18 of the backspan 12 is configured to deform relative to the second and third portions 20, 22 of the backspan 12, wherein the first and second transverse portions 20, 22 of the looped member 18 of the backspan 12 remain substantially linear. Specifically, the deformation of the backspan 12 of the surgical staple 100 is dependent upon a thickness, e.g., "T1," "T2," "T3," etc., of the tissue "T" to be fastened. Initially, in the unformed configuration of the surgical staple 100, the looped member 18 of the backspan 12 includes an uncompressed distance "D1" between an apex 24c of the arcuate portion 24 of the looped member 18 and the second and third portions 20, 22 of the backspan 12, as illustrated in FIG. 2. In some examples, it is contemplated that the apex 24c of the arcuate portion 24 and a mid-portion "M1" (see FIG. 1) of the second portion 20 of the backspan 12 and a mid-portion "M2" (see FIG. 1) of the third portion 22 of the backspan 12 defines an axis "X4-X4" that is substantially parallel to at least one of the first and second legs 14, 16 of the surgical staple 100 and/or substantially perpendicular to the axis "X1-X1" of the second portion 20 of the backspan 12 and the axis "X2-X2" of the third portion 22 of the backspan 12. Upon engagement with the tissue "T," the looped member 18 of the backspan 12 is compressed between the tissue "T" and anvil assembly 1400 (see FIG. 5) of a surgical stapling instrument 1000 such that, the looped member 18 of the backspan 12 defines a compressed distance "D2" between the apex 24c of the arcuate portion 24 of the looped member 18 and the second and third portions 20, 22 of the backspan 12, as illustrated in FIGS. 4A-4C. As the looped member 18 contacts tissue "T" positioned between the first and second legs 14 and the looped member 18 as the staples 100 are deformed, the distance ""D2" decreases an amount that is directly related to the thickness of the tissue "T". More specifically, as the thickness of the tissue increases, the force applied to the looped member 18 by the tissue "T" positioned between the first and second legs 14, 16 and the looped member 18 of the staples 100 as the staples 100 are deformed increases, to increase the amount of deformation of the looped member 18.

FIG. 4A illustrates a staple 100 as the staple 100 is formed in relatively thin tissue "T" having a first thickness "T1". As the staple 100 is deformed about relatively thin tissue "T" having a thickness "T1", the tissue "T" is compressed between the first and second legs 14, 16 and the looped member 18 of the staples 100. As the first and second legs 14, 16 of the staple 100 are deformed into a B-configuration against the anvil assembly 1400 of the surgical stapling instrument 1000 (FIG. 5), the first and second legs 14, 16 push tissue "T" towards and against the looped member 18 of the backspan 12. Because the anvil assembly 1400 is disposed a fixed distance from the cartridge assembly 1300 when the stapling instrument is fired (as described in detail below), all of the tissue "T" must fit between the first and second legs 14, 16 and the looped member 18 of the backspan 12. Thus, where the tissue "T" is relatively thin, the tissue does not apply any substantial forces onto the looped member 18 as the first and second legs 14, 16 are deformed and little or no deformation to the looped member 18 occurs. Thus, the distance "D2" between the apex 24c of the arcuate portion 24 of the looped member 18 and the second and third portions 20, 22 of the surgical staples 100 remains substantially unchanged or only decreases slightly. Referring to FIG. 4B, where the tissue "T" has a moderate thickness of "T2", the tissue "T" requires more space between the first and second legs 14, 16 and the looped member 18 of the backspan 12. Thus, as the first and second legs 14, 16 are deformed against the anvil assembly 1400 and the tissue "T" is pushed towards the looped member 18 of the backspan 12, a greater force is applied to the looped member 18 to cause greater amount of deformation of the looped member 18 of the backspan 12. Thus, the distance "D2" between the apex 24c of the arcuate portion 24 of the looped member 18 and the second and third portions 20, 22 of the backspan 12 of the surgical staple 100 decreases a moderate amount. Similarly, where the tissue "T" has a large thickness of "T3" as shown in FIG. 4C, the tissue "T" requires even more space between the first and second legs 14, 16 and the looped member 18 of the backspan 12 as the staple 100 is deformed. Thus, as the first and second legs 14, 16 are deformed against the anvil assembly 1400 and the tissue "T" is pushed towards the looped member 18 of the backspan 12, an even greater force is applied to the looped member 18 to cause a greater amount of deformation of the looped member 18 of the backspan 12 to further reduce the distance D2" between the apex 24c of the arcuate portion 24 of the looped member 18 and the second and third portions 20, 22 of the backspan 12 of the surgical staple 100.

As illustrated in FIGS. 4A-4C, it is envisioned that the arcuate portion 24 of the looped member 18 of the backspan 12 becomes progressively more linear as the thickness of the tissue "T" increases. The ability of the backspan 12 of the surgical staple 100 to deform in accordance with the relative thickness of the tissue "T" facilitates the use of the presently disclosed staples with tissue having a wider range of thicknesses while providing effective hemostasis.

In order to place the presently disclosed surgical staple 100 in the tissue "T," a surgical apparatus in the form of the surgical stapling instrument 1000 is provided, as illustrated in FIG. 5. The surgical stapling instrument 1000 is approximated and fired similarly to, and in accordance with other known surgical stapling instrument, for example, the surgical stapling instrument disclosed in U.S. Patent No. 8,865,361.

As illustrated in FIG. 5, the surgical stapling instrument 1000 generally includes a handle assembly 1002 with a movable handle 1003a and a stationary handle 1003b, an elongated shaft 1004 extending distally from the handle assembly 1002, and a loading unit 1008 that is coupled to a distal portion of the elongated shaft 1004. In any of the examples disclosed herein, the handle assembly can include, or be attached to, one or more motors, or could be configured to work with a surgical robotic system.

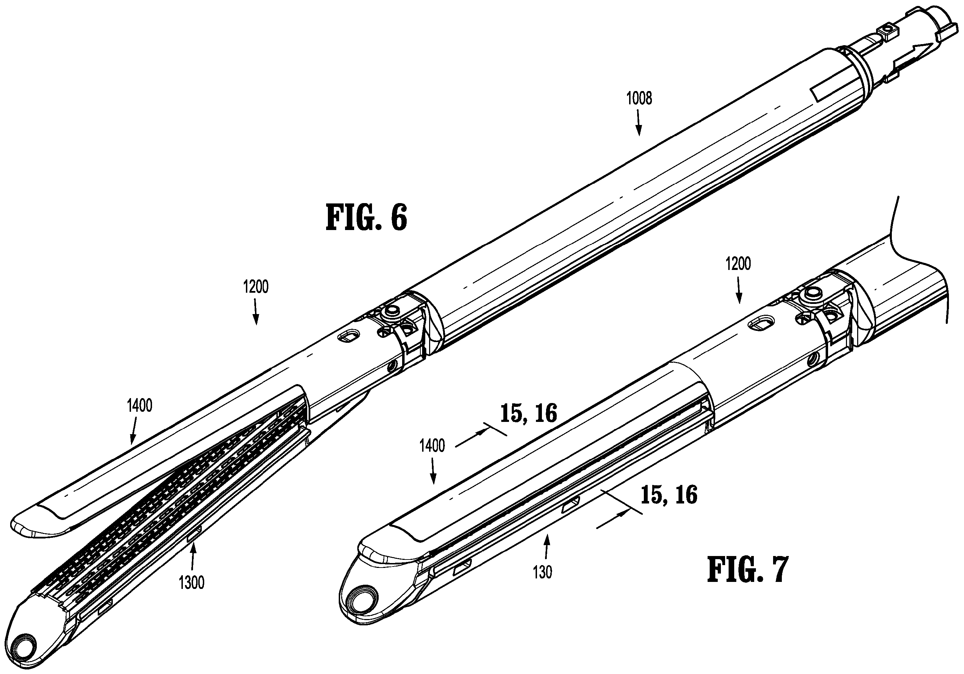

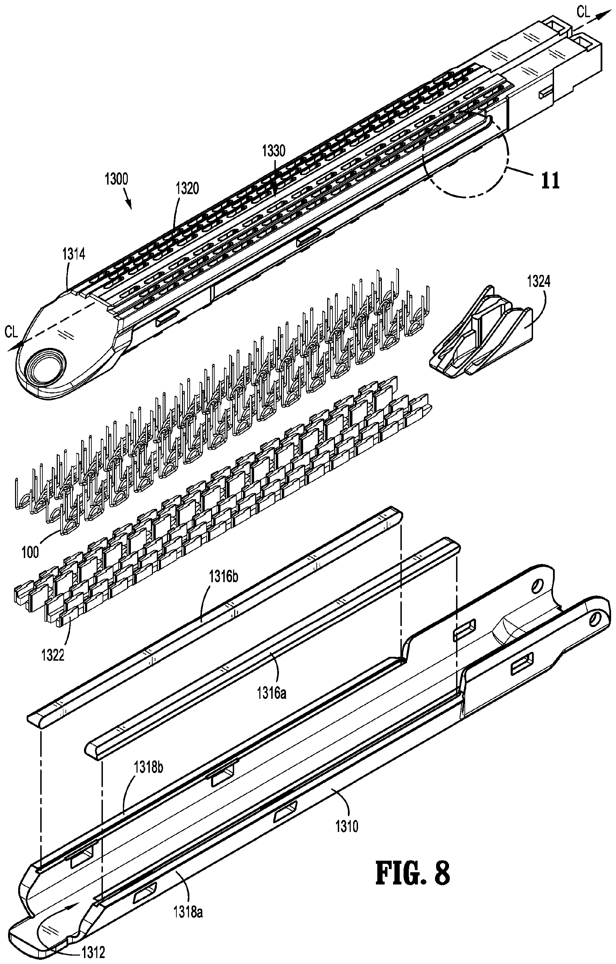

With reference to FIGS. 6-8, the loading unit 1008 of the surgical stapling instrument 1000 includes a tool assembly 1200 having a staple cartridge assembly 1300 housing a plurality of surgical staples 100 (see FIG. 8) and an anvil assembly 1400 movably secured in relation to the staple cartridge assembly 1300 such that the tool assembly 1200 is movable between an open configuration (see FIG. 6) where the staple cartridge assembly 1300 is spaced apart from the anvil assembly 1400, and a clamped configuration (see FIG. 7) where the staple cartridge assembly 1300 and the anvil assembly 1400 are approximated. Alternately, the cartridge assembly 1300 can be movably supported in relation to the anvil assembly 1400.

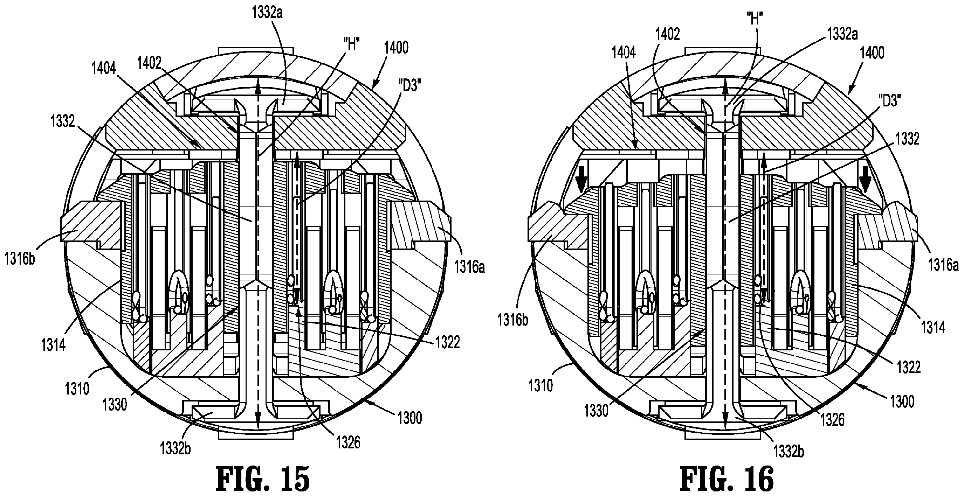

Turning now to FIG. 8, the staple cartridge assembly 1300 includes a carrier 1310 having an elongated support channel 1312. The elongated support channel 1312 is dimensioned and configured to receive a staple cartridge 1314. The staple cartridge assembly 1300 includes a pair of elastic or resilient members 1316a, 1316b that are configured and dimensioned to apply and maintain a constant compressive force to the tissue "T" positioned between the staple cartridge assembly 1300 and the anvil assembly 1400 (see FIGS. 15 and 16) of the tool assembly 1200. In examples, the pair of elastic or resilient members 1316a, 1316b may be configured as two substantially parallel, elongate members that are positioned between the staple cartridge 1314 and a pair of shoulders 1318a, 1318b formed on the carrier 1310, respectively. The pair of elastic members 1316a, 1316b may be attached to, or otherwise disposed on, the pair of shoulders 1318a, 1318b of the carrier 1310, and may be fixedly or releasably attached thereto in alternative examples. The pair of elastic members 1316a, 1316b is configured to compress to accommodate tissues of different thicknesses between the cartridge assembly 1300 and the anvil assembly 1400. For a more detailed description of the construction and operation of an example of the pair of elastic members 1316a, 1316b, reference may be made to U.S. Patent No. 8,152,041.

The staple cartridge 1314 of the staple cartridge assembly 1300 includes a plurality of staple pockets 1320 that are arranged in rows. The plurality of staple pockets 1320 are dimensioned for receiving the plurality surgical staples 100 and a plurality of pushers 1322, as will be detailed below. The staple cartridge assembly 1300 includes an actuation sled 1324 movably supported with in the elongated support channel 1312 of the carrier 1310. During operation, the actuation sled 1324 is configured to advance along the elongated support channel 1312 of the carrier 1310 to sequentially contact the plurality of pushers 1322, such that the plurality of pushers 1322 are displaced within the plurality of staple pockets 1320 to eject the plurality of surgical staples 100 from the plurality of staple pockets 1320 towards the anvil assembly 1400.

As detailed above with reference to FIGS. 1-4C, the axis "X1-X1" of the second portion 20 of the backspan 12 and the axis "X2-X2" of the third portion 22 of the backspan 12" are laterally offset from each other by the offset distance "OD". In order to compensate for the laterally offset orientation of the backspan12 of the surgical staple 100 and ensure that the first and second legs 14, 16 of each of the plurality of surgical staples 100 are longitudinally aligned relative to the other plurality of surgical staples 100 in a corresponding row of surgical staples 100, the plurality of staple pockets 1320 of the staple cartridge 1314 and a plurality of staple seats 1326 of the plurality of pushers 1322 are dimensioned and shaped as detailed below.

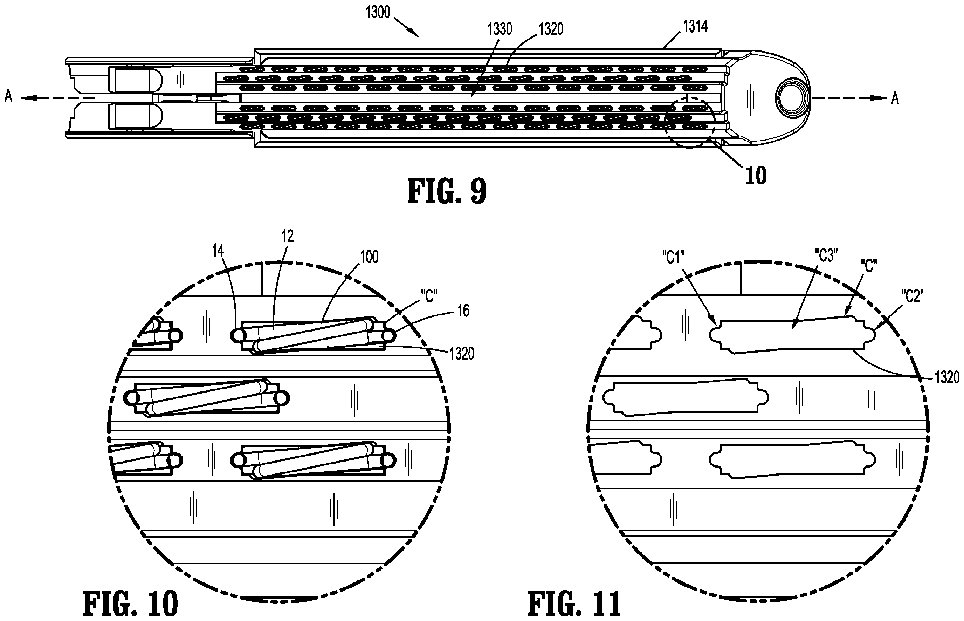

Specifically, with reference to FIGS. 9-11, each of the plurality of staple pockets 1320 of the staple cartridge 1314 includes a contour "C" that corresponds to the orientation of the surgical staple 100 as shown in the top view of the surgical staple 100 in FIG. 3. The contour "C" of the staple pocket 1320 of the staple cartridge 1314 includes a first portion "C1" that is shaped and dimensioned to receive the first leg 14 of the surgical staple 100, a second portion "C2" that is shaped and dimensioned to receive the second leg 16 of the surgical staple 100, and an intermediate portion "C3" positioned between the first portion "C1" and the second portion "C2" that is shaped and dimensioned to receive the backspan 12 of the surgical staple 100. The plurality of staple pockets 1320 of the staple cartridge 1314 are skewingly positioned relative to a longitudinal axis "A-A" of the staple cartridge 1314 such that the first and second legs 14, 16 of each of the plurality of surgical staples 100 are longitudinally aligned relative to the other plurality of surgical staples 100 in the corresponding row of surgical staples 100.

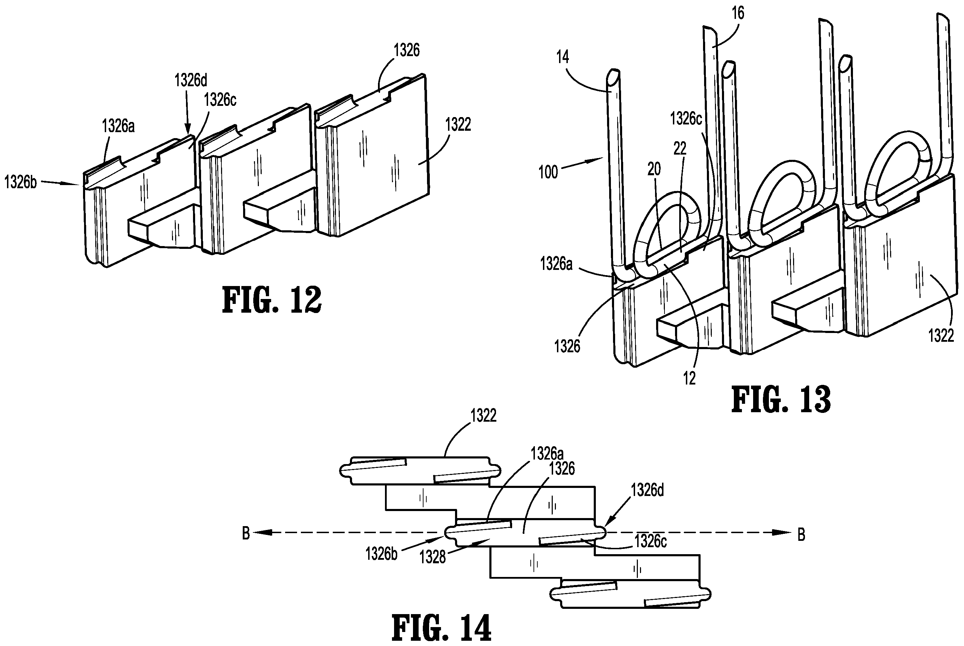

With reference to FIGS. 12-14, each of the plurality of staple seats 1326 of the plurality of pushers 1322 is adapted for releasably receiving the backspan 12 of the surgical staple 100. The staple seat 1326 of the pusher 1322 is skewingly positioned relative to a longitudinal axis "B-B" of the pusher 1322, as illustrated in FIG. 14. It is envisioned that the skewed configuration of the staple seat 1326 of the pusher 1322 is adapted to align the surgical staple 100 with the skewingly positioned staple pocket 1320 of the staple cartridge 1314, as the surgical staple 100 is ejected through the staple pocket 1320 towards the anvil assembly 1400, as illustrated in FIGS. 10, 15, and 16. Specifically, the staple seat 1326 of the pusher 1322 includes a first wall 1326a projecting from a first portion 1326b of the staple seat 1326 and towards the staple pocket 1320 of the staple cartridge 1314, and a second wall 1326c projecting from a second portion 1326d of the staple seat 1326 and towards the staple pocket 1320 of the staple cartridge 1314. The first and second walls 1326a, 1326c of the staple seat 1326 of the pusher 1322 defines a skewed channel 1328 extending between the first and second portions 1326b, 1326d of the staple seat 1326 of the pusher 1322. The skewed channel 1328 of the staple seat 1326 is skewingly positioned relative to the longitudinal axis "B-B" of the pusher 1322.

When the surgical staple 100 is located on the staple seat 1326 of the pusher 1322, the first wall 1326a of the staple seat 1326 is configured to engage the second portion 20 of the backspan 12 and the second wall 1326b of the staple seat 1326 is configured to engage the third portion 22 of the backspan 12 to releasably receive the backspan 12 of the surgical staple 100. Since the skewed channel 1328 of the staple seat 1326 is skewingly positioned relative to the longitudinal axis "B-B" of the pusher 1322, it is envisioned that when the surgical staple 100 is located on the staple seat 1326 of the pusher 1322, the surgical staple 100 is skewingly positioned relative to the longitudinal axis "B-B" of the pusher 1322, as illustrated in FIGS. 13 and 14.

Turning now to FIGS. 8, 9, 15, and 16, in examples, the plurality of staple pockets 1320 are arranged in rows on lateral sides of a knife slot 1330 extending through the staple cartridge 1314 of the staple cartridge assembly 1300. The knife slot 1330 is configured to accommodate movement of a knife 1332, or other such cutting element to sever the tissue "T" (see FIGS. 4A-4C) disposed between the staple cartridge assembly 1300 and the anvil assembly 1400. In examples, the knife slot 1330 may extend along a centerline "CL" of the staple cartridge 314 of the staple cartridge assembly 1300, as illustrated in FIG. 8. Alternatively, the knife slot 1330 may be laterally offset from the centerline "CL" of the staple cartridge 1314 of the staple cartridge assembly 1300.

With reference to FIGS. 15 and 16, the anvil assembly 1400 includes a corresponding knife slot 1402 on a tissue-facing surface 1404 of the anvil assembly 1400 that is configured to accommodate movement of the knife 1332. In examples, the knife 1332 includes an I-beam configuration such that a top portion 1332a of the knife 1332 is movably disposed within the knife slot 1402 of the anvil assembly 1400 and a bottom portion 1332b of the knife 1332 is movably disposed within the knife slot 1330 of the staple cartridge 1314 of the staple cartridge assembly 1300. In any of the examples disclosed herein, the knife can have other shapes, or could be part of or attached to the sled.

With continued reference to FIGS. 15 and 16, the knife 1332 includes a height "H". When the staple cartridge assembly 1300 and the anvil assembly 1400 are approximated and the knife 1332 is translated through the respective knife slots 1330, 1402, the height "H" of the knife 1332 provides for a constant distance "D3" between the anvil assembly 1400 and each of the plurality of staple seats 1326 of the plurality of pushers 1322, regardless of the thickness of the tissue "T" (see FIGS. 4A-4C) disposed between the staple cartridge assembly 1300 and the anvil assembly 1400 and regardless of the positioning of the staple cartridge 1314. The height "H" of the knife 1332 also provides for a maximum tissue gap when the knife 1332 is translated through the respective knife slots 1330, 1402.

Referring now to FIGS. 5-16, in operation, the surgical stapling instrument 1000 is manipulated such that the tissue "T" is disposed between the staple cartridge assembly 1300 and the anvil assembly 1400 with the tool assembly 1200 spaced-apart, in the open configuration (see FIG. 6). The staple cartridge assembly 1300 and the anvil assembly 1400 are then approximated by actuating the movable handle 1003A of the handle assembly 1002 to clamp the tissue "T" disposed between the staple cartridge assembly 1300 and the anvil assembly 1400 such that a compressive force is applied to the tissue "T".

With the tissue "T" securely clamped between the staple cartridge assembly 1300 and the anvil assembly 1400, the surgical stapling instrument 1000 is then fired to eject the plurality of surgical staples 100 by actuating the movable handle 1003A. Upon firing the surgical stapling instrument 1000, the actuation sled 1324 (FIG. 8) advances along the elongated support channel 1312 of the carrier 1310 to sequentially contact the plurality of pushers 1322, such that the plurality of pushers 1322 are displaced within the plurality of staple pockets 1320 to eject the plurality of surgical staples 100 from the plurality of staple pockets 1320 towards the anvil assembly 1400.

The plurality of surgical staples 100 pass through the plurality of staple pockets 1320 of the staple cartridge 1314 (see FIG. 8) and through the tissue "T". After passing through the tissue "T," the plurality of surgical staples 100 engage the tissue-facing surface 1404 of the anvil assembly 1400 and are deformed into the substantially B-staple configuration (see FIGS. 4A-4C). Upon formation within the tissue "T," the plurality of surgical staples 100 maintain a compressive force on the tissue "T" to effect hemostasis.

Sequential firing of the surgical staples 100 continues until the actuation sled 1324 is advanced to a distal end of the staple cartridge 1314, at which time all of the plurality of surgical staples 100 housed the staple cartridge 1314 will have been ejected. The knife 1332 may then be translated through the tool assembly 1200 to form an incision between the rows of stapled tissue "T".

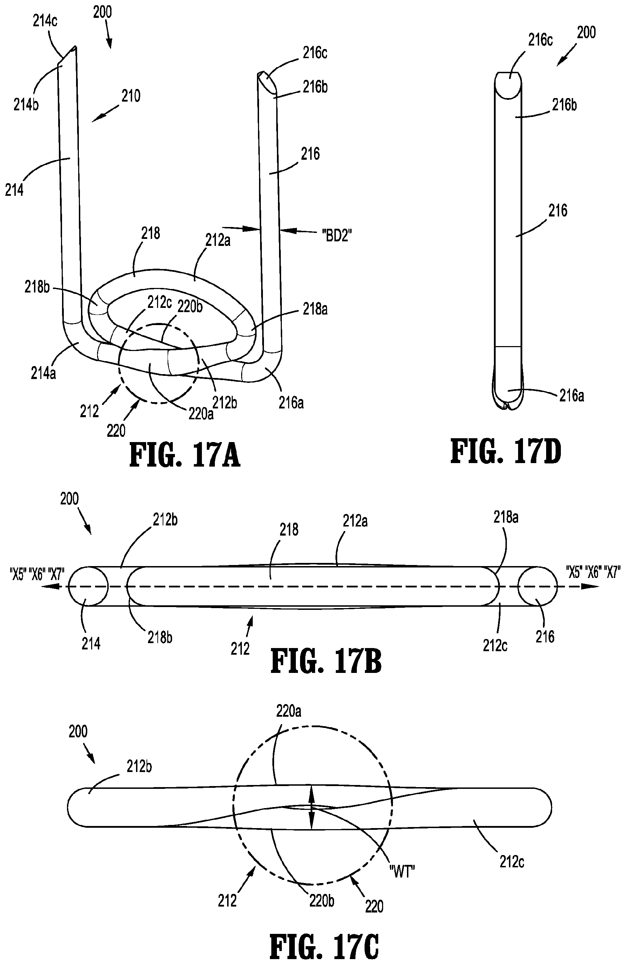

Turning now to FIGS. 17A-17D, an exemplary embodiment of a surgical staple in accordance with the present invention is shown generally as surgical staple 200. The surgical staple 200 is substantially similar to the surgical staple 100 disclosed above (see FIGS. 1-4C) and will therefore be described only to the extent necessary to highlight the differences.

As shown in FIG. 17A, the surgical staple 200 includes a body 210 having a backspan 212, a first leg 214, and a second leg 216. Each of the first and second legs 214, 216 includes a first end portion 214a, 216a, respectively, and a second end portion 214b, 216b, respectively. Each of the second end portions 214b, 216b of the first and second legs 214, 216 includes a tissue-penetrating tip 214c, 216c. The surgical staple 200 has an unformed configuration, as shown in FIGS. 17A, wherein the first leg 214 and the second leg 216 are parallel, or substantially parallel, to one another and spaced a distance from one another. Alternatively, in the unformed configuration, the first and second legs 214, 216 can diverge slightly or converge slightly, etc. in relation to each other.

The backspan 212 includes a first portion 212a including a looped member 218, a second portion 212b that is substantially linear and extends between the first end portion 214a of the first leg 214 and a first end portion 218a of the looped member 218, and a third portion 212c that is substantially linear and extends between the first end portion 216a of the second leg 216 and a second end portion 218b of the looped member 218.

With reference to FIGS. 17A and 17C, according to the invention, the second and third portions 212b, 212c of the backspan 212 defines a crimped region 220 disposed between the first end portion 214a of the first leg 214 and the first end portion 216a of the second leg 216. According to the invention the crimped region 220 includes a first crimped section 220a formed on the second portion 212b of the backspan 212 and a second crimped section 220b formed on the third portion 212c of the backspan 212. According to the invention the first crimped section 220a overlaps with the second crimped section. In an alternative example the first crimped section 220a is axially aligned with the second crimped section 220b. Alternatively, it is contemplated that the crimped region 220 may be defined by a crimped section in only one of the second and third portions 212b, 212c of the backspan 212. In the crimped region 220, the second and/or the third portions 212b, 212c of the backspan 212 are flattened such that a combined wire thickness "WT" of the first and second crimped sections 220a, 220b of the backspan 212 is approximately equal to a diameter "BD2" of the body 210 of the surgical staple 200. In embodiments, the crimped region 220 of the backspan 212 may be formed by compressing the surgical staple 200 between two substantially flat surfaces via any suitable means.

With additional reference to FIG. 17B, forming the crimped region 220 in the surgical staple 200 aligns an axis "X5-X5" of the second portion 212b with an axis "X6-X6" of the third portion 212c. Further, forming the crimped region 220 in the surgical staple 200 aligns an axis "X7-X7" of the first portion 212a with the axis "X5-X5" and the axis "X6-X6" of the second and third portions 212b, 212c of the backspan 212, respectively. As a result, the first leg 214 of the surgical staple 200 is axially aligned with the second leg 216 of the surgical staple 200 such that the surgical staple 200 is provided with a slim profile as shown in FIG. 17D.

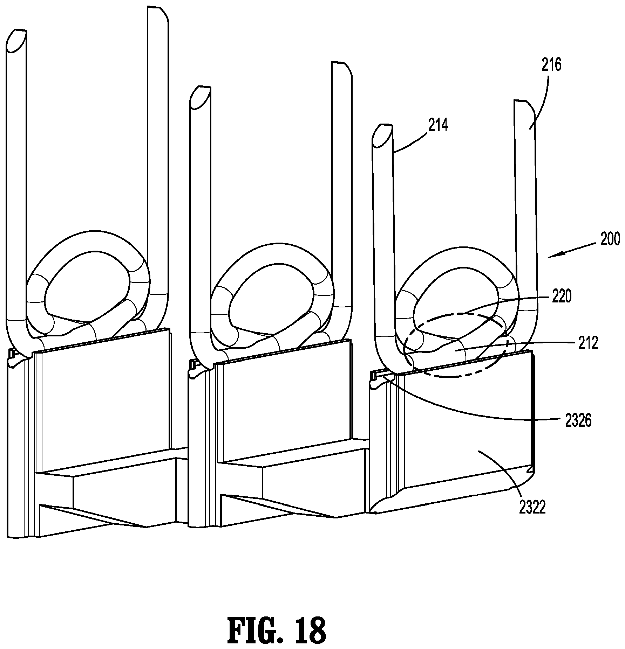

In embodiments, the surgical staple 200 may be used with a known surgical stapling instrument, such as, for example, the surgical stapling instrument disclosed in U.S. Patent No. 5,865,361. With reference to FIG. 18, the surgical stapling instrument disclosed in the '361 patent may include conventional pushers such as, for example, a plurality of pushers 2322. In contrast to the skewed configuration of the plurality of pushers 1322 described above (see FIG. 13), each of the plurality of pushers 2322 includes staple seats 2326 that are linear and configured to support the crimped region 220 of a respective surgical staple 200. The slim profile of the surgical staple 200 (see FIG. 17D) also enables the surgical staple 200 to be received through conventional retention slots of a staple cartridge as disclosed in the '361 patent. Thus, it is contemplated that the surgical staple 200 may be used interchangeably with a conventional surgical staple without requiring modifications to the plurality of pushers 2322 or the staple cartridge as disclosed in the '361 patent.

Referring also to FIGS. 4A-4C, as discussed above in regard to the surgical staple 100, the surgical staple 200 may be positioned adjacent the tissue "T" while in an unformed configuration, and then deformed or fastened onto the tissue such that the surgical staple 200, in a formed configuration, defines a substantially B-shaped staple configuration. In order to place the surgical staple 200 in the tissue "T," a surgical apparatus similar to the surgical stapling instrument 1000 (see FIG. 5) disclosed above, and the surgical stapling instrument disclosed in the '361 patent may be used.

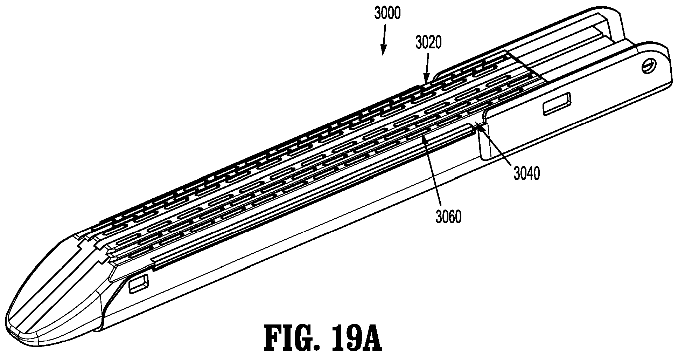

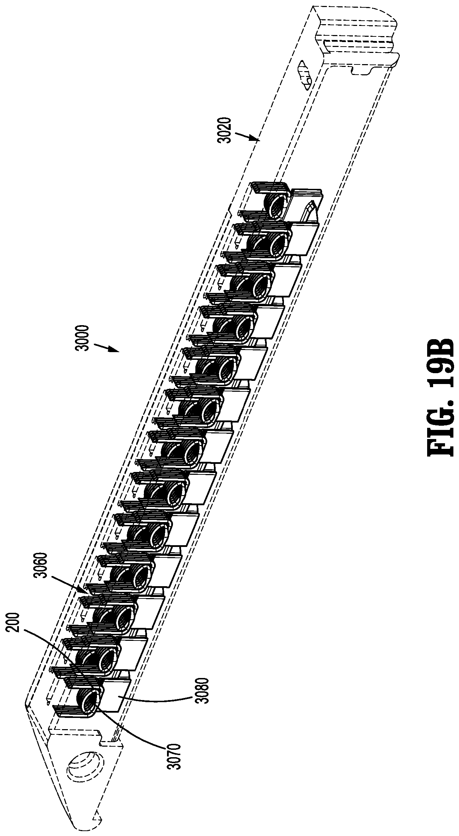

With reference to FIGS. 19A and 19B, the slim profile (see FIG. 17D) of the surgical staple 200 also enables a plurality of surgical staples 200 to be disposed in a stacked orientation, and provided for use with a cartridge assembly 3000 similar to the In-Situ Loaded cartridge assembly disclosed in U.S. Patent No. 9,364,217.

The cartridge assembly 3000 generally includes a first staple cartridge 3020, a second staple cartridge 3040, and a plurality of staple pockets 3060 associated with each of the first and second staple cartridges 3020, 3040. Although Fig. 19B only illustrates the first staple cartridge 3020, each of the first and second staple cartridges 3020, 3040 includes a plurality of staple magazines 3070 and a plurality of pushers 3080 operatively associated with the plurality of staple pockets 3060. Each of the plurality of staple magazines 3070 is configured to receive the plurality of surgical staples 200 such that the plurality of surgical staples 200 is disposed therein in the stacked orientation. Once the plurality of surgical staples 200 is loaded into the plurality of staple magazines 3070, the plurality of pushers 3080 is configured to urge the plurality of surgical staples 200 out through the plurality of staple pockets 3060 as disclosed in '217 patent. It is contemplated that the plurality of surgical staples 200 may be used interchangeably with a plurality of conventional staples as disclosed in the '217 patent without requiring modifications to the cartridge assembly 3000.

In any of the examples disclosed herein, the tool assembly can be incorporated with the elongate portion of the handle assembly. The staple cartridge can be a removable and replaceable assembly, in an instrument with a replaceable loading unit or tool assembly or incorporated tool assembly.

The surgical staples described herein can be utilized in a configuration where the backspan is not intended to be deformed. In certain examples, the backspan can be configured to house a material or object, with a deformable backspan or a backspan that is not deformable. The material or object can include medically useful materials such as a hemostat or sealant, pharmaceuticals such as chemotherapy agents, and even radio-active agents such as brachytherapy particles or seeds. Such materials or objects can be disposed in the opening formed by the looped member or incorporated in a mesh, woven, braided, non-woven material, or a suture, that is disposed in the looped member.

It will be understood that various modifications may be made to the examples disclosed herein. For example, the above described staple may be formed from any of a variety of surgically acceptable materials including titanium, plastics, resorbable materials, etc. Therefore, the above description should not be construed as limiting, but merely as exemplifications of preferred aspects.

## Claims

1. A surgical staple (100, 200) comprising:
a body (10, 210) having a diameter (BD), the body including:
a first leg (14, 214) having a first end portion (14a, 214a) and a second end portion (14b, 214b);
a second leg (16, 216) having a first end portion (16a, 216a) and a second end portion (16b, 216b); and
a backspan (12, 212) including:
a first portion (12a, 212a) having a looped member (18, 218), the looped member including a first end portion (18a, 218a) and a second end portion (18b, 218b);
a second portion (20, 212b) extending between the first end portion (14a, 214a) of the first leg (14, 214) and the first end portion (18a, 218a) of the looped member (18, 218);
a third portion (22, 212c) extending between the first end portion (16a, 216a) of the second leg (16, 216) and the second end portion (18b, 218b) of the looped member (18, 218); and
a crimped region (220) including a first crimped section (220a) defined on the second portion (212b) of the backspan (212) and a second crimped section (220b) defined on the third portion (212c) of the backspan (212) wherein a combined wire thickness (WT) of the second and third portions (212b, 212c) of the backspan (212) in the crimped region (220) is about equal to the diameter (BD) of the body (10, 210) of the surgical staple (100, 200), **characterised in that** the first crimped section (220a) of the second portion (212b) of the backspan (212) overlaps with the second crimped section (220b) of the third portion (212c) of the backspan (212).

2. The surgical staple (100, 200) according to any preceding claim wherein the looped member of the backspan defines a first axis that extends between the first end portion of the looped member and the second end portion of the looped member; preferably wherein the second portion of the backspan defines a second axis and the third portion of the backspan defines a third axis, the first axis of the looped member being parallel with the second axis of the second portion and the third axis of the third portion; preferably still wherein the second axis of the second portion of the backspan is laterally aligned with the third axis of the third portion of the backspan such that the first leg and the second leg of the surgical staple are axially aligned.

3. The surgical staple (100, 200) according to claim 2, wherein the looped member of the backspan includes an apex (24), the second portion (20) of the backspan (12) includes a first mid-portion (M1), and the third portion (22) of the backspan (12) includes a second mid-portion (M2), the apex (24) of the looped member (18) and the first and second mid-portions (M1, M2) of the second and third portions of the backspan (20, 22) defining an axis that is perpendicular to the axis (X1-X1) of the second portion (20) of the backspan (12) and the axis (X2-X2) of the third portion (22) of the backspan (12).

4. The surgical staple (100) according to claim 3, wherein the surgical staple (100) includes an unformed configuration and a formed configuration, wherein in the unformed configuration of the surgical staple (100), the apex (24) of the looped member (18) of the backspan (12) is spaced apart from each of the second and third portions (20, 22) of the backspan (12) a first distance, and wherein in the formed configuration of the surgical staple (100), the apex (24) of the looped member (18) of the backspan (12) is spaced apart from each of the second and third portions (20, 22) of the backspan (12) a second distance less than the first distance.

5. The surgical staple (100) according to claim 4, wherein the backspan (12) of the surgical staple (100) is positioned to engage tissue such that the second distance between the apex (24) of the looped member (18) of the backspan (12) and each of the second and third portions (20, 22) of the backspan (12) decreases as a thickness of tissue engaged by the backspan (12) increases; and/or wherein in the formed configuration of the surgical staple (100), the first leg (14) of the surgical staple (100) is positioned on a first lateral side (24a) of the looped member (18) and the second leg (16) of the surgical staple (100) is positioned on a second lateral side (24b) of the looped member (18) opposite the first lateral side (24a)of the looped member (18).

6. A staple cartridge assembly (1300) housing a plurality of surgical staples (100, 200) according to any preceding claim.

## Patentansprüche

1. Chirurgische Klammer (100, 200), umfassend:
einen Körper (10, 210) mit einem Durchmesser (BD), wobei der Körper Folgendes aufweist:
einen ersten Schenkel (14, 214) mit einem ersten Endabschnitt (14a, 214a) und einem zweiten Endabschnitt (14b, 214b),
einen zweiten Schenkel (16, 216) mit einem ersten Endabschnitt (16a, 216a) und einem zweiten Endabschnitt (16b, 216b), und
eine hintere Spanne (12, 212), die Folgendes aufweist:
einen ersten Abschnitt (12a, 212a) mit einem geschlungenen Glied (18, 218), wobei das geschlungene Glied einen ersten Endabschnitt (18a, 218a) und einen zweiten Endabschnitt (18b, 218b) aufweist,
einen zweiten Abschnitt (20, 212b), der sich zwischen dem ersten Endabschnitt (14a, 214a) des ersten Schenkels (14, 214) und dem ersten Endabschnitt (18a, 218a) des geschlungenen Glieds (18, 218) erstreckt,
einen dritten Abschnitt (22, 212c), der sich zwischen dem ersten Endabschnitt (16a, 216a) des zweiten Schenkels (16, 216) und dem zweiten Endabschnitt (18b, 218b) des geschlungenen Glieds (18, 218) erstreckt, und
einen gebördelten Bereich (220), der einen ersten gebördelten Abschnitt (220a), der an dem zweiten Abschnitt (212b) der hinteren Spanne (212) definiert ist, und einen zweiten gebördelten Abschnitt (220b), der an dem dritten Abschnitt (212c) der hinteren Spanne (212) definiert ist, aufweist, wobei eine kombinierte Drahtdicke (WT) des zweiten und des dritten Abschnitts (212b, 212c) der hinteren Spanne (212) in dem gebördelten Bereich (220) ungefähr gleich dem Durchmesser (BD) des Körpers (10, 210) der chirurgischen Klammer (100, 200) ist, **dadurch gekennzeichnet, dass** sich der erste gebördelte Abschnitt (220a) des zweiten Abschnitts (212b) der hinteren Spanne (212) mit dem zweiten gebördelten Abschnitt (220b) des dritten Abschnitts (212c) der hinteren Spanne (212) überlappt.

2. Chirurgische Klammer (100, 200) nach einem der vorhergehenden Ansprüche, wobei das geschlungene Glied der hinteren Spanne eine erste Achse definiert, die sich zwischen dem ersten Endabschnitt des geschlungenen Glieds und dem zweiten Endabschnitt des geschlungenen Glieds erstreckt, vorzugsweise wobei der zweite Abschnitt der hinteren Spanne eine zweite Achse definiert und der dritte Abschnitt der hinteren Spanne eine dritte Achse definiert, wobei die erste Achse des geschlungenen Glieds parallel zu der zweiten Achse des zweiten Abschnitts und der dritten Achse des dritten Abschnitts verläuft, weiter bevorzugt wobei die zweite Achse des zweiten Abschnitts der hinteren Spanne seitlich auf die dritte Achse des dritten Abschnitts der hinteren Spanne ausgerichtet ist, so dass der erste Schenkel und der zweite Schenkel der chirurgischen Klammer axial aufeinander ausgerichtet sind.

3. Chirurgische Klammer (100, 200) nach Anspruch 2, wobei das geschlungene Glied der hinteren Spanne einen Scheitel (24) aufweist, der zweite Abschnitt (20) der hinteren Spanne (12) einen ersten mittleren Abschnitt (M1) aufweist und der dritte Abschnitt (22) der hinteren Spanne (12) einen zweiten mittleren Abschnitt (M2) aufweist, wobei der Scheitel (24) des geschlungenen Glieds (18) und der erste und der zweite mittlere Abschnitt (M1, M2) des zweiten und des dritten Abschnitts der hinteren Spanne (20, 22) eine Achse definieren, die senkrecht zu der Achse (X1 - X1) des zweiten Abschnitts (20) der hinteren Spanne (12) und der Achse (X2 - X2) des dritten Abschnitts (22) der hinteren Spanne (12) verläuft.

4. Chirurgische Klammer (100) nach Anspruch 3, wobei die chirurgische Klammer (100) eine ungeformte Konfiguration und eine geformte Konfiguration aufweist, wobei der Scheitel (24) des geschlungenen Glieds (18) der hinteren Spanne (12) in der ungeformten Konfiguration der chirurgischen Klammer (100) von jeweils dem zweiten und dem dritten Abschnitt (20, 22) der hinteren Spanne (12) mit einem ersten Abstand beabstandet ist und wobei der Scheitel (24) des geschlungenen Glieds (18) der hinteren Spanne (12) in der geformten Konfiguration der chirurgischen Klammer (100) von jeweils dem zweiten und dem dritten Abschnitt (20, 22) der hinteren Spanne (12) mit einem zweiten Abstand beabstandet ist, der kleiner als der erste Abstand ist.

5. Chirurgische Klammer (100) nach Anspruch 4, wobei die hintere Spanne (12) der chirurgischen Klammer (100) dazu positioniert ist, Gewebe in Eingriff zu nehmen, so dass der zweite Abstand zwischen dem Scheitel (24) des geschlungenen Glieds (18) der hinteren Spanne (12) und jeweils dem zweiten und dem dritten Abschnitt (20, 22) der hinteren Spanne (12) so abnimmt, wie eine Dicke von Gewebe, das von der hinteren Spanne (12) in Eingriff genommen wird, zunimmt, und/oder wobei der erste Schenkel (14) der chirurgischen Klammer (100) in der geformten Konfiguration der chirurgischen Klammer (100) an einer ersten lateralen Seite (24a) des geschlungenen Glieds (18) positioniert ist und der zweite Schenkel (16) der chirurgischen Klammer (100) an einer der ersten lateralen Seite (24a) des geschlungenen Glieds (18) gegenüberliegenden zweiten lateralen Seite (24b) des geschlungenen Glieds (18) positioniert ist.

6. Klammermagazinanordnung (1300), in der eine Vielzahl von chirurgischen Klammern (100, 200) nach einem der vorhergehenden Ansprüche untergebracht sind.

## Revendications

1. Agrafe chirurgicale (100, 200) comprenant :
un corps (10, 210) ayant un diamètre (BD), le corps comprenant :
une première branche (14, 214) ayant une première partie d'extrémité (14a, 214a) et une seconde partie d'extrémité (14b, 214b) ;
une seconde branche (16, 216) ayant une première partie d'extrémité (16a, 216a) et une seconde partie d'extrémité (16b, 216b) ;et
une barre transversale (12, 212) comprenant :
une première partie (12a, 212a) ayant un élément en boucle (18, 218), l'élément en boucle comprenant une première partie d'extrémité (18a, 218a) et une seconde partie d'extrémité (18b, 218b) ;
une deuxième partie (20, 212b) s'étendant entre la première partie d'extrémité (14a, 214a) de la première branche (14, 214) et la première partie d'extrémité (18a, 218a) de l'élément en boucle (18, 218) ;
une troisième partie (22, 212c) s'étendant entre la première partie d'extrémité (16a, 216a) de la seconde branche (16, 216) et la seconde partie d'extrémité (18b, 218b) de l'élément en boucle (18, 218) ; et
une région sertie (220) comprenant une première section sertie (220a) définie sur la deuxième partie (212b) de la barre transversale (212) et une deuxième section sertie (220b) définie sur la troisième partie (212c) de la barre transversale (212), une épaisseur de fil combinée (WT) des deuxième et troisième parties (212b, 212c) de la barre transversale (212) dans la région sertie (220) étant environ égale au diamètre (BD) du corps (10, 210) de l'agrafe chirurgicale (100, 200), **caractérisée en ce que** la première section sertie (220a) de la deuxième partie (212b) de la barre transversale (212) chevauche la deuxième section sertie (220b) de la troisième partie (212c) de la barre transversale (212).

2. Agrafe chirurgicale (100, 200) selon l'une quelconque des revendications précédentes, l'élément en boucle de la barre transversale définissant un premier axe qui s'étend entre la première partie d'extrémité de l'élément en boucle et la seconde partie d'extrémité de l'élément en boucle ; de préférence, la deuxième partie de la barre transversale définissant un deuxième axe et la troisième partie de la barre transversale définissant un troisième axe, le premier axe de l'élément en boucle étant parallèle au deuxième axe de la deuxième partie et au troisième axe de la troisième partie ; de préférence encore, le deuxième axe de la deuxième partie de la barre transversale étant aligné latéralement avec le troisième axe de la troisième partie de la barre transversale de sorte que la première branche et la seconde branche de l'agrafe chirurgicale sont alignées axialement.

3. Agrafe chirurgicale (100, 200) selon la revendication 2, l'élément en boucle de la barre transversale comprenant un sommet (24), la deuxième partie (20) de la barre transversale (12) comprenant une première partie centrale (M1), et la troisième partie (22) de la barre transversale (12) comprenant une deuxième partie centrale (M2), le sommet (24) de l'élément en boucle (18) et les première et deuxième parties centrales (M1, M2) des deuxième et troisième parties de la barre transversale (20, 22) définissant un axe qui est perpendiculaire à l'axe (X1-X1) de la deuxième partie (20) de la barre transversale (12) et à l'axe (X2-X2) de la troisième partie (22) de la barre transversale (12).

4. Agrafe chirurgicale (100) selon la revendication 3, l'agrafe chirurgicale (100) comprenant une configuration non formée et une configuration formée, dans la configuration non formée de l'agrafe chirurgicale (100), le sommet (24) de l'élément en boucle (18) de la barre transversale (12) étant espacé de chacune des deuxième et troisième parties (20, 22) de la barre transversale (12) d'une première distance, et, dans la configuration formée de l'agrafe chirurgicale (100), le sommet (24) de l'élément en boucle (18) de la barre transversale (12) étant espacé de chacune des deuxième et troisième parties (20, 22) de la barre transversale (12) d'une seconde distance inférieure à la première distance.

5. Agrafe chirurgicale (100) selon la revendication 4, la barre transversale (12) de l'agrafe chirurgicale (100) étant positionnée pour venir en prise avec le tissu de sorte que la seconde distance entre le sommet (24) de l'élément en boucle (18) de la barre transversale (12) et chacune des deuxième et troisième parties (20, 22) de la barre transversale (12) diminue à mesure que l'épaisseur du tissu mis en prise par la barre transversale (12) augmente ; et/ou, dans la configuration formée de l'agrafe chirurgicale (100), la première branche (14) de l'agrafe chirurgicale (100) étant positionnée sur un premier côté latéral (24a) de l'élément en boucle (18) et la seconde branche (16) de l'agrafe chirurgicale (100) étant positionnée sur un deuxième côté latéral (24b) de l'élément en boucle (18) opposé au premier côté latéral (24a) de l'élément en boucle (18).

6. Ensemble de cartouche d'agrafes (1300) logeant une pluralité d'agrafes chirurgicales (100, 200) selon l'une quelconque des revendications précédentes.
